# EUROPEAN PATENT APPLICATION

(11) **EP 3 438 659 A1**
(43) Date of publication of application: **06.02.2019**
(21) Application number: 18182077.0
(22) Date of filing: 06.07.2018
(51) Int. Cl.: G01N 33/20, F27B 5/06, F27B 5/16, G01N 31/12

(54) **ANALYSIS APPARATUS**

(30) Priority: 12.07.2017 JP 2017136610; 12.07.2017 JP 2017136612; 11.12.2017 JP 2017237256
(71) Applicant: Horiba, Ltd., Kyoto-city, Kyoto 601-8510 (JP)
(72) Inventor: HIRANO, Akihiro, Kyoto, 601-8510 (JP); YAMADA, Takahiro, Kyoto, 601-8510 (JP); YONESHIGE, Hidenaru, Kyoto, 601-8510 (JP); INOUE, Takahito, Kyoto, 601-8510 (JP)
(74) Representative: Isarpatent

(57) **Abstract**

Provided is an analysis apparatus capable of analyzing a deflagrative specimen with accuracy. The analysis apparatus includes: a vessel for containing a specimen; a heating furnace to be loaded with the vessel; and an analyzer that analyzes analysis target gas produced from the specimen heated and combusted in the heating furnace. In addition, the analysis apparatus further includes: a combustion supporting gas supply flow path for supplying combustion supporting gas used for the combustion of the specimen to the heating furnace; and a combustion control mechanism adapted to regulate the flow rate of the combustion supporting gas to be supplied to the heating furnace from the combustion supporting gas supply flow path and control the combustion of the specimen.

## Description

### Technical Field

The present invention relates to an analysis apparatus such as an elemental analysis apparatus that analyzes elements such as carbon (C) and sulfur (S) contained in a specimen such as steel, nonferrous metal, or ceramic.

### Background Art

As disclosed in, for example, Patent Literature 1, this sort of analysis apparatus is adapted to load a specimen into a heating furnace to heat it, as well as to supply combustion supporting gas necessary for combustion, such as O₂, to the heating furnace to combust the specimen, and analyze analysis target gas resulting from the combustion by an analyzer such as NDIR.

However, in the case of a deflagrative specimen, problems may occur, such as ones that immediately after starting combustion, the specimen is exploded and scattered in the heating furnace and for example, part of it is attached to a lower temperature part of the heating furnace and fails to be sufficiently combusted, causing a measurement error, and that a remaining specimen piece adversely affects the measurement of next another specimen. In addition, since the specimen is suddenly intensely combusted, the amount of analysis target gas introduced into the analyzer suddenly varies in a short period time, and therefore the response of the analyzer cannot sufficiently follow it, causing the problem of an increase in measurement error.

### Citation List

### Patent Literatures

Patent Literature 1: Japanese Unexamined Patent Application Publication No. 61-274259

### Summary of Invention

### Technical Problem

Therefore, the present invention has been made in order to solve such problems, and a main intended object thereof is to provide an analysis apparatus capable of analyzing a deflagrative specimen with accuracy.

### Solution to Problem

That is, the analysis apparatus according to the present invention is one including: a vessel for containing a specimen; a heating furnace to be loaded with the vessel; and an analyzer that analyzes analysis target gas produced from the specimen heated and combusted in the heating furnace. In addition, the analysis apparatus further includes: a combustion supporting gas supply flow path for supplying combustion supporting gas used for the combustion of the specimen to the heating furnace; and a combustion control mechanism adapted to regulate the flow rate of the combustion supporting gas to be supplied to the heating furnace from the combustion supporting gas supply flow path and control the combustion of the specimen.

Even when the specimen to be loaded into the heating furnace is a deflagrative one that is explosively intensely combusted, such as coal, the analysis apparatus as described above is capable of suppressing the combustion by decreasing the flow rate of the combustion supporting gas and thereby preventing the specimen from being scattered by explosion, and can therefore surely combust the specimen, thus making it possible to contribute to improving measurement accuracy and preventing the occurrence a problem due to a remaining specimen piece. Also, suppressing the combustion enables a time variation in the amount of the analysis target gas introduced into the analyzer to be reduced, and therefore the response of the analyzer can sufficiently follow the variation to consequently reduce a measurement error.

On the other hand, in the case of a specimen not having deflagration, the flow rate of the combustion supporting gas can be regulated to supply the combustion supporting gas as usual, and therefore measurement time can be prevented from being unnecessarily prolonged.

As one specific embodiment of the combustion control mechanism, one including: a bypass flow path branching from the combustion supporting gas supply flow path; and opening level regulating means adapted to regulate the opening level of the bypass flow path, and adapted to regulate the flow rate of the combustion supporting gas to be supplied to the heating furnace by flowing part of the combustion supporting gas flowing through the combustion supporting gas supply flow path to the bypass flow path can be cited.

In the above-described configuration, in order to make it possible to more finely regulate the flow rate of the combustion supporting gas to be supplied to the heating furnace in multiple steps, it is preferable that the bypass flow path is one including multiple branch bypass flow paths that are provided in parallel and respectively have predetermined resistances, and the opening level regulating means is one including on-off valves respectively provided in the branch bypass flow paths.

The flow rate of gas to be introduced into the analyzer is preferably always substantially constant in terms of ensuring measurement accuracy. For this purpose, it is only necessary that the bypass flow path branching from the combustion supporting gas supply flow path is provided in parallel with the heating furnace and communicatively connects to the analyzer.

This is because in such a configuration, only by making substantially constant the flow rate of the combustion supporting gas to be supplied to the combustion supporting gas supply flow path as a source, even when the flow rate of the combustion supporting gas to be supplied to the heating furnace is changed for combustion control, the change is finally introduced into the analyzer through the bypass flow path and consequently the flow rate of the gas flowing through the analyzer is always substantially constant.

Meanwhile, usually, when calibrating this sort of analysis apparatus (more specifically, offset calibration), the combustion supporting gas is supplied to the analyzer as zero gas, and the output value of the analyzer is set to zero. When doing this, the combustion supporting gas is not heated.

In addition, it should be appreciated that as the combustion supporting gas, a gas species not interfering with the measurement of the analysis target gas by the analyzer is used. For example, when measuring carbon (C) and sulfur (S) components in a specimen, analysis target gas contains CO₂ and SO₂ that are combusted materials of the components, and in this case, as the combustion supporting gas, oxygen (O₂) insensitive to the measurement of CO₂ and SO₂ is used.

However, if a slight amount of impurities such as hydrocarbons (HC) is contained in the combustion supporting gas, the combustion supporting gas is not heated at the time of calibration and the contained hydrocarbons exist in the combustion supporting gas without change. Accordingly, although the analyzer whose sensitivity is set for CO₂ and SO₂ does not detect the hydrocarbons, the hydrocarbons contained in the combustion supporting gas is heated in the heating furnace at the time of analysis, and combusted to convert to H₂O and CO₂, and the analyzer detects them, causing a measurement error.

However, measures like providing a gas purifier to remove the hydrocarbons in the combustion supporting gas through the gas purifier may cause new problems of limiting analytical sensitivity to the purification accuracy of the gas purifier and also increasing the cost and size of the analyzer due to the introduction of the gas purifier.

In order to solve such problems at once, it is only necessary to supply the combustion gas after heat treatment to the analyzer at the time of calibration. In doing so, since the impurities in the combustion supporting gas have been combusted at the time of calibration as well as at the time of analysis, even when the analyzer is sensitive to the combusted impurities, by setting the output value of the analyzer at the time of the calibration to zero, a measurement error due to the combusted impurities can be cancelled. In addition, since it is only necessary to add a mechanism adapted to heat the combustion supporting gas, cost and size are not unreasonably increased.

Specifically, when the analysis apparatus has a branch flow path for introducing the combustion supporting gas into the analyzer without the heating furnace for, for example, calibration or the above-described combustion control, it is only necessary to provide heating means in the branch flow path.

Further specific embodiments of the analysis apparatus is one further including: a shut-off valve that shuts off the combustion supporting gas introduced to the heating furnace to wholly introduce the combustion supporting gas flowing through the combustion supporting gas supply flow path to the branch flow path; and heating means adapted to heat the combustion supporting gas flowing through the branch flow path. In addition, an offset correction value for the analyzer is determined with use of an output result of the analyzer in a state where the shut-off valve operates and the combustion supporting gas is wholly introduced into the analyzer through the branch flow path.

Also, in the case of the analysis apparatus not including a branch flow path, it is only necessary to evacuate the heating furnace and then heat the heating furnace, and at the time of calibration, introduce the combustion supporting gas heated in the heating furnace into the analyzer.

As a specific embodiment of the heating furnace, one including: a furnace body for containing the vessel; and an electric resistor for generating heat by current application to heat the furnace body can be cited.

Usually, a piping member for introducing the analysis target gas led out of the heating furnace into the analyzer is designed to have a certain length so as to prevent the heat effect of the heating furnace on the analyzer. Also, in order to collect dust discharged from the heating furnace, a dust filter is provided on the analyzer side of the piping member.

However, such a mechanism cannot prevent the dust from being attached to the piping member, and therefore it is necessary to clean and replace not only the dust filter but the piping member.

In order to solve this problem, it is only necessary that a dust removing mechanism is configured to be attached continuously with a gas lead-out port for leading out the analysis target gas to the analyzer, and the piping member is configured to be attached to the subsequent stage of the dust removing mechanism.

In addition, it is further preferable that the dust removing mechanism is one including a filter for collecting dust, and a casing for holding the filter inside, and the casing has a horizontal pipe part adapted to circulate the analysis target gas in a horizontal direction and hold the filter, and includes a concave part below the filter in the horizontal pipe part.

In such a configuration, since the dust removing mechanism is attached continuous with the gas lead-out port, i.e., continuous with the heating furnace, the temperature of the dust removing mechanism itself can be kept high to some extent. For this reason, the attachment of SO₂ as one of measurement targets as a result of dissolution in moisture condensed in the dust removing mechanism, and the like can be prevented, thus making it possible to suppress a reduction in measurement accuracy due to the attachment. Further, since the casing is configured to be formed with the concave part below the dust filter in the horizontal pipe part, dust once collected by the filter and then falling from it can be collected in the concave part provided below, and therefore dust can be easily cleaned.

In addition, a conventional analysis apparatus includes only one mensurative heating furnace for combusting a specimen to produce analysis target gas, and in order to continuously analyze multiple specimens, it is necessary to take out a combusted specimen from the mensurative heating furnace and weigh a new specimen to load it again into the mensurative heating furnace for each of the specimens. Further, after loading the new specimen, it takes time to combust it and produce analysis target gas, which is very inefficient.

In order to solve such a problem, the analysis apparatus according to the present invention may include: multiple heating furnaces; and a heating furnace selecting mechanism adapted to selectively communicatively connect any of the heating furnaces to the analyzer.

Since such a configuration includes the multiple heating furnaces each capable of combusting a specimen and producing analysis target gas, during the combustion of a specimen in one heating furnace and the subsequent analysis of analysis target gas by the analyzer, a specimen can be combusted in another heating furnace to prepare analysis target gas to be analyzed next. Then, the heating furnace selecting mechanism communicatively connects the heating furnace in which the analysis target gas is prepared to the analyzer, and thereby the analysis of the next analysis target gas can be promptly started. In doing so, it is possible to eliminate the time and effort required to take out an analyzed specimen from a mensurative heating furnace and to weigh a new specimen to load it into the mensurative heating furnace, and the time required to, after loading the new specimen, combust it and produce analysis target gas, and therefore multiple specimens can be continuously analyzed with efficiency.

As specific embodiments of the heating furnace selecting mechanism of the analysis apparatus of the present invention include one including: a plurality of gas lead-out flow paths whose start points communicates with the respective heating furnaces and whose end points communicates with the analyzer; and on-off valves for opening/closing the respective gas lead-out flow paths.

Preferably, a specific embodiment of the analysis apparatus of the present invention is one making the plurality of heating furnaces loaded with vessels containing specimens simultaneously perform heating operation and making the heating furnace selecting mechanism communicate only one of the furnaces with the analyzer.

In the analysis apparatus of the present invention, it is preferable that on upper stream sides than the on-off valves in the gas lead-out flow paths, openable/closable gas discharge flow paths for discharging gas flowing through the gas lead-out flow paths are connected.

In such a configuration, for example, when baking or the like of a vessel is performed in a heating furnace not communicating with the analyzer, produced gas can be discharged without retention in the heating furnace. For this reason, when a specimen is combusted for analysis using the heating furnace after the baking, a measurement error caused by the gas produced by the baking can be reduced.

Also, usually, when measuring free carbon contained in a specimen using this sort of analysis apparatus, it is necessary to combust the specimen at a low temperature of approximately 800 °C to 900 °C over time and thereby produce analysis target gas from the specimen. However, in the case of combusting at the low temperature over time as described, the amount of the analysis target gas introduced into the analyzer per unit time is small, and therefore the intensity of a signal outputted from the analyzer is low, causing the problem of deteriorating an S/N ratio. In addition, since the analysis is performed over a long time, even when the offset correction of the analyzer was made before the analysis, a shift in zero point from a baseline increases during measurement, causing the problem of deteriorating measurement accuracy.

In order to solve such problems, it is only necessary that the internal temperature of the heating furnace is changeable between a predetermined first temperature range and a second temperature range lower than the first temperature range, and in a state where the internal temperature of the heating furnace is in the second temperature range, an on-off valve in a gas lead-out flow path of the heating furnace is configured to be switched from a closed state to an opened state after a predetermined time has passed since the specimen was loaded into the heating furnace.

In such a configuration, by setting the internal temperature of the heating furnace to the second temperature range, only analysis target gas derived from free carbon can be produced from a specimen. In addition, by retaining such analysis target gas in the gas lead-out flow path for a predetermined time without promptly introducing the analysis target gas into the analyzer, and then bringing the on-off valve in the gas lead-out flow path into the opened state, the retained analysis target gas can be introduced into the analyzer at once. For this reason, the amount of the analysis target gas introduced into the analyzer per unit time is increased to increase the intensity of a signal outputted from the analyzer, and thereby the S/N ratio can be improved. Further, since analysis can be performed in a relatively short time after the on-off valve has been opened, the analysis can be performed in a stage where the shift in zero point of the analyzer from the baseline is relatively small, and therefore the measurement accuracy of the free carbon can be improved.

Also, an analysis method of the present invention is one that in a heating furnace, combusts a specimen contained in a vessel, and with use of an analyzer, analyzes analysis target gas resulting from the combustion, and the analysis method includes: heating combustion supporting gas used to combust the specimen; introducing the heated combustion supporting gas into the analyzer; and determining an offset correction value for the analyzer with use of an output result of the analyzer in a state where the heated combustion supporting gas is introduced.

In such a configuration, since impurities in the combustion supporting gas have been combusted even at the time of calibration of the analyzer as well as at the time of analysis, even when the analyzer is sensitive to the combusted impurities, by setting the output value of the analyzer to zero at the time of the calibration, a measurement error due to the combusted impurities can be cancelled.

### Advantageous Effects of Invention

According to the present invention, even in the case of a deflagrative specimen, the combustion control mechanism can control the combustion of the specimen to prevent the specimen from being scattered by explosion or the like, and therefore the specimen can be surely combusted, thus making it possible to contribute to improving measurement accuracy and preventing the occurrence of a problem due to a remaining specimen piece.

### Brief Description of Drawings

FIG. 1 is a diagram schematically illustrating the configuration of an analysis apparatus in the present embodiment;
FIG. 2 is a cross-sectional view schematically illustrating the configuration of a heating furnace in the same embodiment;
FIG. 3 is a cross-sectional view schematically illustrating the configuration of a first dust removing mechanism in the same embodiment;
FIG. 4 is a diagram illustrating examples of an output result from an analyzer in the same embodiment; and
FIG. 5 is a diagram schematically illustrating the configuration of an analysis apparatus in a variation.

### Description of Embodiments

In the following, one embodiment of the analysis apparatus according to the present invention will be described with reference to the drawings.

### <Configuration of analysis apparatus 100>

An analysis apparatus 100 of the present embodiment is one that heats and combusts a specimen such as metal, and from the resulting gas, analyzes elements such as carbon (C) and sulfur (S) contained in the specimen.

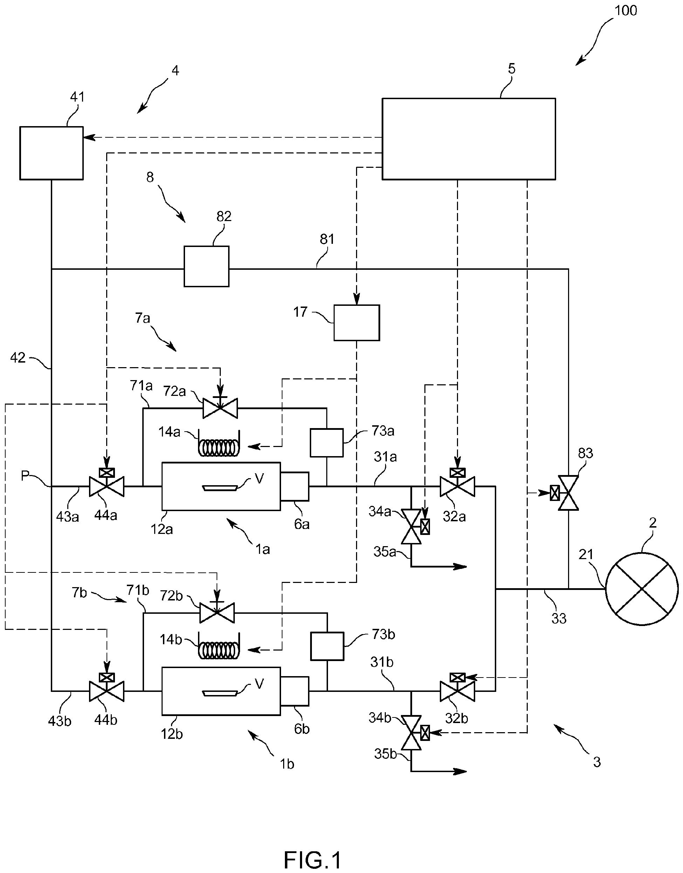

Specifically, as illustrated in FIG. 1, the analysis apparatus 100 includes: vessels V each for containing a specimen; a first heating furnace 1a and a second heating furnace 1b to be loaded with the vessels V; a first dust removing mechanism 6a and a second dust removing mechanism 6b respectively provided just after the first heating furnace 1a and the second heating furnace 1b; an analyzer 2 that analyzes analysis target gas resulting from combusting a specimen; a heating furnace selecting mechanism 3 adapted to selectively communicatively connect any of the first heating furnace 1a and the second heating furnace 1b to the analyzer 2; a combustion supporting gas supplying mechanism 4 adapted to supply combustion supporting gas into the first heating furnace 1a and the second heating furnace 1b; and a control part 5 that controls the first heating furnace 1a, the second heating furnace 1b, the heating furnace selecting mechanism 3, and the combustion supporting gas supplying mechanism 4.

The respective parts will be described below. Note that in the description below, among the components illustrated in the drawings, multiple components having symbols whose numerals are the same but alphabets are different mean having the same configurations (e.g., the first heating furnace 1a and the second heating furnace 1b have the same configuration), and therefore description thereof may be omitted.

A vessel V is one that can contain a specimen inside, and placed in the first heating furnace 1a or the second heating furnace 1b. Specifically, the vessel V is a porcelain combustion boat.

The first heating furnace 1a and the second heating furnace 1b are ones that inside them, bake the vessels V not containing the specimens or heat the vessels V containing the specimens to combust them.

As illustrated in FIG. 1, the first heating furnace 1a and the second heating furnace 1b respectively include: furnace bodies 12a and 12b for containing the vessels V; and electric resistors 14a and 14b, and are provided with a current control circuit 17 adapted to apply current to the electric resistors 14a and 14b.

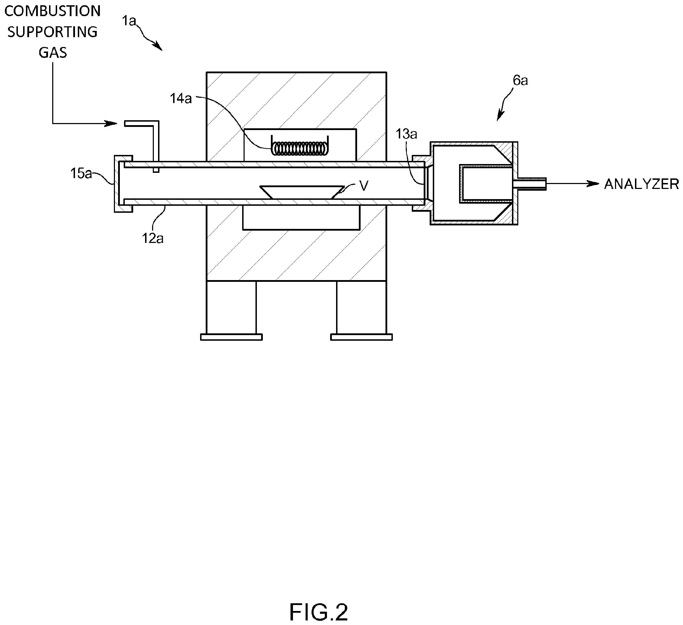

The detailed configuration of the first heating furnace 1a will be described here using FIG. 2. Note that although only the configuration of the first heating furnace 1a is illustrated as a representative example here, the second heating furnace 1b also has the same configuration.

The furnace body 12a is a ceramic molding of a circular pipe shape, and can contain the vessel V inside. One end part of the furnace body 12a is provided with a cap 15a, and by removing the cap 15a, the vessel V can be moved into or out of the furnace body 12a. The other end part of the furnace body 12a is provided with a gas lead-out port 13a for leading out the analysis target gas produced from the specimen to the analyzer 2.

Around the furnace body 12a, the electric resistor 14a is provided. The electric resistor 14a is one for generating heat by current application to heat the furnace body 12a.

As illustrated in FIG. 2, the first dust removing mechanism 6a is attached to the gas lead-out port 13a of the first heating furnace 1a, continuous with the gas lead-out port 13a.

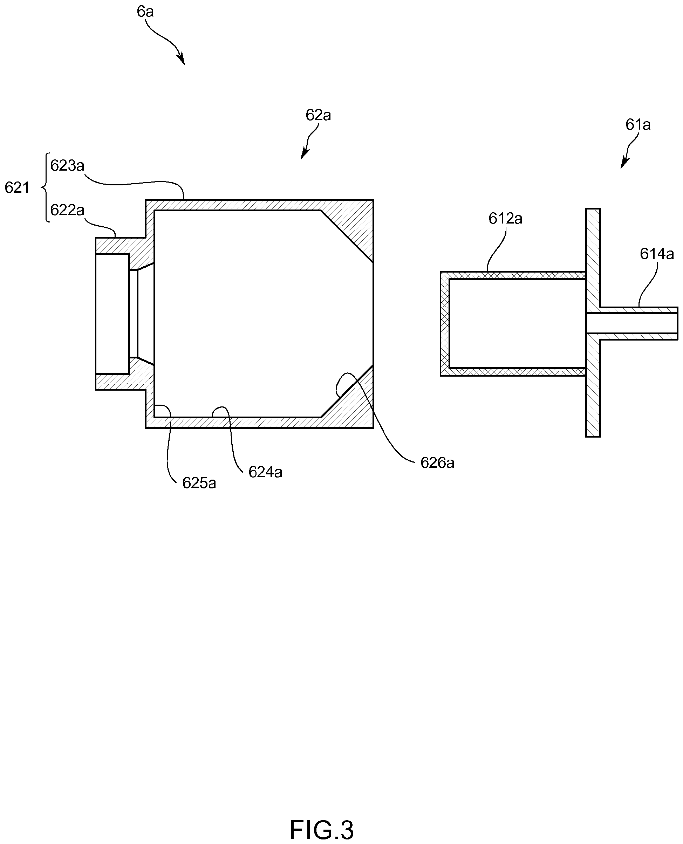

The first dust removing mechanism 6a is one for removing dust and the like contained in the analysis target gas. Specifically, as illustrated in FIG. 3, the first dust removing mechanism 6a includes: a first member 61a adapted to collect dust; and a second member 62a adapted to contain dust that cannot be collected by the first member 61a and falls.

FIG. 3 illustrates a state where the first dust removing mechanism 6a is decomposed and separated into the first member 61a and the second member 62a. As illustrated, the first member 61a is configured to be attachable and detachable with respect to the second member 62a. The respective members will be described.

The first member 61a is one including: a filter 612a for collecting dust; and a flange 614a that holds the filter 612 a, as well as forms a flow path for the analysis target gas. The filter 612a in the present embodiment is a mesh-like sintered metal filter made of stainless steel, and a substantially cylindrical one having an axis in the horizontal direction. The flange 614a mainly includes a disc-like member coaxial with the filter 612a and a pipe member protruding from the disc-like member toward the analyzer 2 side.

The second member 62a includes: a first horizontal pipe part 622a connecting to the gas lead-out port 13a; and a second horizontal pipe part 623a adapted to circulate the analysis target gas in the horizontal direction and hold and contain the filter 612a inside. The first horizontal pipe part 622a and the second horizontal pipe part 623a are both substantially cylindrical ones having axes in the horizontal direction. The first horizontal pipe part 622a and the second horizontal pipe part 623a are provided continuous with each other in a direction in which the analysis target gas flows, and function as a casing of the first dust removing mechanism 6a. In the end part 626a of the second horizontal pipe part 623a on the analyzer 2 side, an opening is formed, and the filter 612a of the first member 61a is adapted to be fitted into the opening.

Below the filter 612a in the second horizontal part 623a, the inner wall of the second horizontal pipe part 623a forms a concave part 624a. For this reason, dust once collected by the filter 612a and then falling from it can be collected in the concave part 624a, and therefore dust can be easily cleaned.

In addition, the first horizontal pipe part 622a and the second horizontal pipe part 623 a are formed coaxial with each other, and the second horizontal pipe part 623a is configured to be larger in inside diameter than the first horizontal pipe part 622a. In doing so, the connection part between the first horizontal pipe part 622a and the second horizontal pipe part 623a is formed with a level difference 625a, and the level difference 625a forms a side wall of the concave part 624a. As a result, dust once collected by the filter 612a and then falling from it is more easily collected, and therefore dust can be more easily cleaned.

The end part 626a of the second horizontal pipe part 623a on the analyzer 2 side is formed in a tapered shape so that an opening formed by the inner surface thereof gradually decreases in size toward the analyzer 2 side. Accordingly, when detaching the first member 61a from the second member 62a to clean the inside of the second member 62a, dust is unlikely to be caught by the inner wall of the second horizontal pipe part 623a, and therefore dust can be easily discharged.

Also, since the first member 61a is one attachable and detachable with respect to the second member 62a as described above, by detaching the respective members from each other, the inner surfaces of both the members can be easily cleaned.

The gas analyzer 2 is one that analyzes the analysis target gas produced in the first or second heating furnace 1a or 1b to obtain the contents of respective components contained in the specimen. In the present embodiment, the gas analyzer 2 is one adapted to perform the analysis using a nondispersive infrared absorption method (NDIR method). Specifically, the gas analyzer 2 has an unillustrated nondispersive infrared detector, and obtains the contents of carbon (C), sulfur (S), and the like contained in the specimen by detecting CO₂, CO, SO₂, and the like contained in the gas led out of the heating furnace 1a or 1b. In the present embodiment, the gas analyzer 2 makes an offset correction using reference gas such as oxygen, and then detects the analysis target gas introduced from a gas introduction port 21 to measure signal intensity with time.

The heating furnace selecting mechanism 3 is one adapted to selectively communicatively connect any of the first heating furnace 1a and the second heating furnace 1b to the analyzer 2. Specifically, the heating furnace selecting mechanism 3 includes: a first gas lead-out flow path 31a; a second gas lead-out flow path 31b; an on-off valve 32a for opening/closing the first gas lead-out flow path 31a; an on-off valve 32b for opening/closing the second gas lead-out flow path 31b; and an analysis target gas introduction flow path 33.

The first gas lead-out flow path 31a is one for leading out the analysis target gas from the first heating furnace 1a. The start point of the first gas lead-out flow path 31a is configured to be connected to the flange 614a of the first dust removing mechanism 6a and thereby communicatively connect to the furnace body 12a of the first heating furnace 1a. Also, the end point of the first gas lead-out flow path 31a is configured to be connected to the on-off valve 32a and thereby communicatively connect to the gas introduction port 21 of the analyzer 2 via the on-off valve 32a. The second gas lead-out flow path 31b is also configured in the same manner.

The analysis target gas introduction flow path 33 is one for introducing the analysis target gas led out of the first heating furnace 1a or the second heating furnace 1b to the analyzer 2. The start point of the analysis target gas introduction flow path 33 is configured to be connected to the on-off valves 32a and 32b and thereby communicatively connect to the first heating furnace 1a and the second heating furnace 1b. Also, the end point of the analysis target gas introduction flow path 33 is configured to be connected to the gas introduction port 21 of the analyzer 2 and thereby communicatively connect to the analyzer 2.

From the first gas lead-out flow path 31a, a first gas discharge flow path 35a for discharging gas flowing through the first gas lead-out flow path 31a branches. In the first gas discharge flow path 35a, an on-off valve 34a is provided, and by bringing the on-off valve 34a into the opened state, the gas in the first heating furnace 1a and the first gas lead-out flow path 31a can be discharged. A second gas discharge flow path 35b is also configured in the same manner.

The combustion supporting gas supplying mechanism 4 is one adapted to supply the combustion supporting gas into the first heating furnace 1a and into the second heating furnace 1b. In the present embodiment, oxygen is used as the combustion supporting gas. The combustion supporting gas supplying mechanism 4 is specifically one including a combustion supporting gas supply source 41 and a combustion supporting gas supply flow path 42.

The combustion supporting gas supply source 41 is one for sending the combustion supporting gas to the combustion supporting gas supply flow path 42, and specifically one including an oxygen cylinder, a regulator attached to the oxygen cylinder, and the like. The combustion supporting gas supply source 41 is configured to be capable of keeping the inside of the combustion supporting gas supply flow path 42 at a constant pressure equal to or more than atmospheric pressure.

The combustion supporting gas supply glow path 42 is one for supplying the combustion supporting gas led out of the combustion supporting gas supply source 41 into the first heating furnace 1a and the second heating furnace 1b. The start point of the combustion supporting gas supply flow path 42 is configured to communicatively connect to the combustion supporting gas supply source 41, and the end point of the combustion supporting gas supply flow path 42 is configured to be connected to the furnace bodies 12a and 12b of the first and second heating furnaces 1a and 1b and thereby communicatively connect into the furnace bodies 12a and 12b. The combustion supporting gas supply flow path 42 is one including: a first combustion supporting gas introduction flow path 43a that branches at a branch point P and is for introducing the combustion supporting gas to the first heating furnace 1a; and a second combustion supporting gas introduction flow path 43b for introducing the combustion supporting gas to the second heating furnace 1b (i.e., the combustion supporting gas introduction flow paths 43a and 43b are parts of the combustion supporting gas supply flow path 42).

The first combustion supporting gas introduction flow path 43a is one for supplying the combustion supporting gas to the first heating furnace 1a. The start point of the first combustion supporting gas introduction flow path 43a is connected to the branch point P to thereby communicatively connect to the combustion supporting gas supply source 41 via the combustion supporting gas supply flow path 42, and the end point is connected to and communicatively connects to the furnace body 12a of the first heating furnace 1a. In the first combustion supporting gas introduction flow path 43, an on-off valve 44a for opening/closing the first combustion supporting gas introduction flow path 43a is provided. The second combustion supporting gas introduction flow path 43b is also configured in the same manner.

Further, in this embodiment, a combustion control mechanism 7a or 7b adapted to control combustion of the specimen by regulating the flow rate of the combustion supporting gas to be supplied from the combustion supporting gas introduction flow path 43a or 43b to the heating furnace 1a or 1b is provided for each of the heating furnaces.

The combustion control mechanism 7a includes: a bypass flow path 71a that branches from the first combustion supporting gas introduction flow path 43a and communicatively connects to the analyzer 2; and opening level regulating means adapted to regulate the opening level of the bypass flow path 71a.

The bypass flow path 71a is here configured to connect to the first gas lead-out flow path 31a at the end point thereof and thereby communicatively connect to the gas introduction port 21 of the analyzer 2 via the first gas lead-out flow path 31a and the analysis target gas introduction flow path 33.

The opening level regulating means is here a flow rate regulation valve 72a, and the opening level thereof can be continuously regulated by a signal from the control part 5.

The combustion control mechanism 7 also includes heating means 73a configured to be capable of heating the combustion supporting gas flowing through the bypass flow path 71a to the same temperature range as that of the heating furnace 1a. The heating means 73a is here one having a heater and the like attached around the bypass flow path 71a.

The combustion control mechanism 7b is also configured in the same manner as the combustion control mechanism 7a.

Still further, in this embodiment, one combustion supporting gas heating mechanism 8 adapted to heat the combustion supporting gas supplied from the combustion supporting gas supply source 41 is provided for the multiple heating furnaces 1a and 1b.

The combustion supporting gas heating mechanism 8 includes: a branch flow path 81 that branches from the combustion supporting gas supply flow path 42 and communicatively connects to the analyzer 2; and heating means 82 configured to be capable of heating the combustion supporting gas flowing through the branch flow path 81 to the same temperature range as that of the heating furnaces.

The branch flow path 81 is here configured to be connected to the upper stream side than the branch point P in the combustion supporting gas supply flow path 42 at the start point thereof, and thereby communicatively connect to the combustion supporting gas supply source 41 via the combustion supporting gas supply flow path 42. Also, the branch flow path 81 is configured to be connected to the analysis target gas introduction flow path 33 at the end point thereof and thereby communicatively connect to the gas introduction port 21 of the analyzer 2 via the analysis target gas introduction flow path 33.

The heating means 82 is here configured to have a heater and the like attached around the branch flow path 81.

The control part 5 is one that controls opening/closing of the on-off valves 32a, 32b, 34a, 34b, 44a, 44b, 72a, and 72b, and of control valves 83a and 83b to change flow paths for the combustion supporting gas and/or the analysis target gas, and controls the current control circuit 17 to regulate the current to be applied to the electric resistors. Structurally, the control part 5 is a so-called computer circuit including a CPU, an internal memory, an I/O buffer circuit, an A/D converter, and the like. In addition, the CPU and its peripheral devices cooperate in accordance with a control program stored in a predetermined area of the internal memory to thereby fulfill functions as the control part 5.

Next, an example of a method for analyzing a specimen using the analysis apparatus 100 of the present embodiment will be described in detail. As will be described below, when analyzing a specimen using the analysis apparatus 100 of the present embodiment, the specimen is combusted in the first heating furnace 1a (or the second heating furnace 1b), then the resulting analysis target gas is retained for a predetermined time, and the analysis target gas is supplied to the analyzer 2 to perform the analysis. At the same time, while the analysis using the first heating furnace 1a (or the second heating furnace 1b) is being performed, a specimen is combusted in the second heating furnace 1b (or the first heating furnace 1a), and the resulting analysis target gas is retained for a predetermined time to make preparations so that analysis using the second heating furnace 1b (or the first heating furnace 1a) can be promptly started. Then, after the analysis using the first heating furnace 1a (or the second heating furnace 1b) has been finished, the analyzer 2 is calibrated, and then the analysis target gas retained in the second heating furnace 1b (or the first heating furnace 1a) is supplied to the analyzer 2 to perform the analysis.

Specifically, first, an operator operates an unillustrated operation panel attached to the analysis apparatus 100 to perform a heating operation so that the internal temperatures of the heating furnaces fall within a first temperature range. The control part 5 having sensed the operation starts to heat the first heating furnace 1a and the second heating furnace 1b.

Then, on a temperature monitor (not illustrated) that is attached to the analysis apparatus 100 and displays the internal temperatures of the furnaces, the operator confirms that the internal temperatures of the heating furnaces 1a and 1b have fallen within the first temperature range from 1300 °C to 1500 °C, and opens the cap of the heating furnace to load a vessel containing a specimen into the first heating furnace 1a.

The operator operates the operation panel to perform an operation of start heating the specimen in the first heating furnace 1a. The control part 5 having sensed the operation brings the on-off valve 44a into the opened state from the closed state. This allows the combustion supporting gas to be supplied into the first heating furnace 1a through the first combustion gas introduction flow path 43a, and the specimen is combusted. Analysis target gas resulting from the combustion is retained in the first heating furnace 1a and the first gas lead-out flow path 31a.

Subsequently, after a predetermined setting retention time has passed since the on-off valve 44a was brought into the opened state, the control part 5 brings the on-off valve 32a into the opened state from the closed state. As a result, the analysis target gas retained in the first heating furnace 1a and the first gas lead-out flow path 31a is introduced into the analyzer 2 to start analysis.

Note that the setting retention time may be a time longer or shorter than the time required to completely combust the specimen. That is, the analysis target gas may be introduced into the analyzer 2 after the specimen has been completely combusted or while the specimen is being combusted.

After a predetermined analysis required time has passed since the on-off valve 32a was brought into the opened state, the control part 5 brings the on-off valves 32a and 44a into the closed state from the opened state. As a result, the supply of the combustion supporting gas into the first heating furnace 1a is shut off, and the combustion and analysis of the specimen are finished.

Note that the predetermined analysis required time is a time after the on-off valve 32a has been brought into the opened state, and may be a time (e.g., 60 seconds) specified by the operator in advance. Alternatively, the predetermined analysis required time may be a time after the on-off valve 32a has been brought into the opened state, and the time necessary for the intensity of a signal outputted by the analyzer 2 to reduce to a predetermined percentage or less (e.g., 1/100 or less) of peak intensity.

In this manner, the specimen is combusted in the first heating furnace 1a and the analysis target gas is analyzed, and during the combustion and analysis, preparation operation for analyzing the next specimen can be performed in the second heating furnace 1b. Specifically, operations of loading a vessel containing the specimen into the second heating furnace 1b and starting to combust the specimen in the second heating furnace 1b are performed. The control part 5 having sensed the operations brings the on-off valve 44b into the opened state from the closed state. This allows the combustion supporting gas to be supplied into the second heating furnace 1b through the second combustion supporting gas introduction flow path 43b, and the specimen is combusted. Analysis target gas resulting from the combustion is retained in the second heating furnace 1b and the second gas lead-out flow path 31b. After the analysis using the first heating furnace 1a has been finished and the on-off valve 32a has been brought into the closed state from the opened state, an operation of performing the analysis using the second heating furnace 1b is performed. The control part 5 having sensed the operation brings the on-off valve 32b into the opened state from the closed state, and the analysis target gas retained in the second heating furnace 1b is supplied to the analyzer 2 to start the analysis.

After that, as described above, preparation operation for analyzing the next specimen can be performed in the first heating furnace 1a.

That is, in the analysis apparatus 100 of the present embodiment, while using one heating furnace 1a (or 1b) to analyze a specimen, a specimen can be combusted in the other heating furnace 1b (or 1a) to make preparations for analysis.

Next, the calibration operation of the analyzer 2 of the present analysis apparatus 100 will be described.

In this embodiment, just before the analysis using each of the above-described heating furnaces 1a and 1b, the calibration of the analyzer 2 is inevitably automatically performed. For the calibration, the branch flow path 81 is used.

A detailed description will be given below. Just before analysis target gas is flowed to the analyzer 2, the on-off valves 44a and 44b for introducing the combustion supporting gas to the respective heating furnaces 1a and 1b are closed (accordingly, the on-off valves 44a and 44b for introducing the combustion supporting gas function as shut-off valves in claims), and an on-off valve 83 is opened, so that the whole of the combustion supporting gas flows through the branch flow path 81 and is in a state of being introduced into the analyzer 2. In addition, at this time, the heating means 82 also operates, and the combustion supporting gas is heated to the first temperature range when passing through the heating means 82.

As described above, with only the combustion supporting gas once heated introduced into the analyzer 2, the analyzer 2 is subjected to the calibration operation, more specifically, zero point calibration.

The above is the calibration operation.

Next, the operation of the analysis apparatus 100 when the specimen is deflagrative will be described. Note that the description of the operation is based on the assumption that one heating furnace 1a (or 1b) containing the specimen is in an operating state; the supply of the combustion supporting gas to the other heating furnace 1b (or 1a) used for baking is in a stopped state; and the supply of the combustion supporting gas to the branch flow path 81 is in a stopped state. That is, it is assumed that the on-off valve 44b (or 44a) for introducing the combustion supporting gas and the on-off valve 83 are in the stopped state.

In the case of the deflagrative specimen, an operator operates the operation panel to set a flow rate so as to reduce the flow rate of the combustion supporting gas to be introduced into the heating furnace 1a (or 1b) containing the specimen as compared with a normal specimen case. The control part 5 senses this to control the flow rate regulation valve 72a (or 72b). The combustion supporting gas flows divided into the heating furnace 1a (or 1b) and the bypass flow path 71a (or 71b), and therefore by operating the flow rate regulation valve 72a (or 72b) in an opening direction to increase the flow rate of the combustion supporting gas flowing through the bypass flow path 71a (orb71b), the flow rate of the combustion supporting gas supplied to the heating furnace 1a (or 1b) is decreased.

As described above, after setting the flow rate regulation valve 72a (or 72b), the on-off valve 44a (or 44b) for introducing the combustion supporting gas into the heating furnace 1a (or 1b) used for analysis is opened, and consequently, the combustion supporting gas flows to the heating furnace 1a (or 1b) to start combusting the specimen.

That is, the combustion of the specimen is suppressed by the reduction in flow rate of the combustion supporting gas, and therefore even in the case of the deflagrative specimen, neither suddenly combustion nor consequent explosion occurs.

Subsequently, as described above, after the predetermined setting retention time has passed, the on-off valve 32a (or 32b) for leading out the combustion supporting gas from the heating furnace 1a (or 1b) is opened, and the analysis target gas is introduced into the analyzer 2 and analyzed.

Note that since the combustion supporting gas also functions as carrier gas for sending the analysis target gas from the heating furnace 1a (or 1b) to the analyzer 2, after the setting retention time has passed, the control part 5 controls the flow rate regulation valve 72a (72b) to increase the flow rate of the combustion supporting gas to the heating furnace 1a (or 1b), and thereby the transfer time of the analysis target gas to the analyzer 2 is shortened.

In addition, in this case, the heating means 73a (or 73b) is adapted to heat the combustion supporting gas flowing through the bypass flow path 71a (or 71b) to the same temperature range as that of the heating furnace 1a (or 1b), and combust impurities such as HC in the combustion supporting gas flowing through the bypass flow path 71a (or 71b) as in the heating furnace 1a (or 1b). In doing so, a measurement error is prevented from occurring in the analyzer 2 having been subjected to the zero point calibration in the above-described manner.

Even when a specimen to be loaded into a heating furnace is deflagrative, the analysis apparatus 100 according to the present embodiment configured as described above can prevent the specimen from being scattered due to explosion by decreasing the flow rate of the combustion supporting gas to suppress combustion, and therefore surely combust the specimen, thus making it possible to contribute to improving measurement accuracy and preventing the occurrence of a problem due to a remaining specimen piece. In addition, suppressing the combustion enables a time variation in the amount of the analysis target gas introduced into the analyzer to be reduced, and therefore the response of the analyzer can sufficiently follow the variation to consequently reduce a measurement error.

On the other hand, in the case of a specimen not having deflagration, the flow rate of the combustion supporting gas can be regulated to supply the combustion supporting gas as usual, and therefore measurement time can be prevented from being unnecessarily prolonged.

Further, since when calibrating the analyzer 2, the heated combustion supporting gas is supplied to the analyzer 2, impurities in the combustion supporting gas have been combusted even at the time of calibration as well as at the time of analysis, and therefore even when the analyzer 2 is sensitive to combusted impurities, by setting the output value of the analyzer 2 at the time of calibration to zero, a measurement error due to the combusted impurities can be cancelled. In addition, it is only necessary to add a mechanism adapted to heat the combustion supporting gas, and therefore neither cost nor size is significantly increased.

Further, according to this embodiment, the following effects can also be obtained.

Since the analysis apparatus 100 includes the two heating furnaces each capable of combusting a specimen to produce analysis target gas, i.e., the first heating furnace 1a and the second heating furnace 1b, during the combustion of a specimen in one heating furnace 1a (or 1b) and the subsequent analysis of analysis target gas, a specimen can be combusted in the other heating furnace 1b (or 1a) to prepare analysis target gas to be analyzed next. Then, the heating furnace selecting mechanism 3 communicatively connects the heating furnace 1b (or 1a) in which the analysis target gas is prepared to the analyzer 2, and thereby the analysis of the next analysis target gas can be promptly started. In doing so, it is possible to eliminate the time and effort required to take out an analyzed specimen from a mensurative heating furnace and to weigh a new specimen to load it to the mensurative heating furnace, and the time required to, after loading the new specimen, combust it to produce analysis target gas, and therefore multiple specimens can be continuously analyzed with efficiency.

Also, since the analysis apparatus 100 according to the present embodiment is such that on the upper stream sides than the on-off valves 32a and 32b in the gas lead-out flow paths 31a and 31b, the openable/closable gas discharge flow paths 35a and 35b for discharging gas flowing through the gas lead-out flow paths 31a and 31b are connected, gas produced in a heating furnace not communicatively connecting to the analyzer 2 when, for example, baking a vessel can be discharged without retention in the heating furnace. For this reason, when combusting a specimen using the heating furnace to perform analysis after the baking, a measurement error caused by the gas produced by the baking can be reduced.

Further, since the analysis apparatus 100 of the present embodiment is such that the dust removing mechanisms 6a and 6b are attached continuous with the gas lead-out ports 13a and 13b of the heating furnaces 1a and 1b, the temperatures of the dust removing mechanisms 6a and 6b themselves can be kept high to some extent. For this reason, it is possible to suppress the occurrence of the problem of changing an SO₂ amount in analysis target gas to reduce measurement accuracy as a result of dissolution of SO₂ as one of measurement targets in moisture condensed in a dust removing mechanism 6a or 6b.

### <Other embodiments>

Note that the present invention is not limited to the above-described embodiment.

The above-described analysis method using the analysis apparatus 100 is one adapted to start combusting a specimen with both of the on-off valves 32 remaining in the closed state, and bring a relevant on-off valve 32 into the opened state to introduce analysis target gas into the analyzer 2, but not limited to this. The analysis method may be configured to start combusting a specimen with the on-off valves 32 remaining in the opened state and promptly introduce analysis target gas into the analyzer 2.

Also, the above-described analysis method using the analysis apparatus 100 is one adapted to be capable of, while analyzing a specimen using one heating furnace 1a (or 1b), combusting a specimen in the other heating furnace 1b (or 1a) and retaining the resulting analysis target gas to prepare analysis, but not limited to this. In another embodiment, while a specimen is being analyzed using one heating furnace 1a (or 1b), baking for containing a specimen may be performed in the other heating furnace 1b (or 1a).

Specifically, while analysis is being performed using the first heating furnace 1a, an operator operates the operation panel with an empty vessel V placed in the second heating furnace 1b and performs an operation of starting baking in the second heating furnace 1b. The control part 5 having sensed this brings the on-off valves 34b and 44b into the opened state from the closed state. In doing so, the combustion supporting gas is supplied into the second heating furnace 1b through the second combustion supporting gas introduction flow path 43b to bake the vessel V, as well as to produce gas derived from impurities attached on the vessel. The gas is discharged through the second lead-out flow path 31b and the second gas discharge flow path 35b.

The analysis method using the analysis apparatus 100 of the above-described embodiment is one adapted to combust a specimen with the internal temperature of a heating furnace 1a or 1b in the first temperature range from 1300 °C to 1500 °C and analyze the specimen, but, not limited to this.

An analysis apparatus 100 of another embodiment may be one adapted to analyze a specimen with the internal temperature of a heating furnace 1a or 1b in a predetermined second temperature range, for example, from 800 °C to 900 °C, which is lower than the first temperature range.

By using a heating furnace 1a or 1b with the internal temperature of the heating furnace 1a or 1b in such a second temperature range, only analysis target gas derived from free carbon contained in a specimen can be measured by the analyzer 2.

Also, in an analysis apparatus 100 of another embodiment, the furnace body 12a of the first heating furnace 1a may be a silica tube and the furnace body 12b of the second heating furnace 1b may be a ceramic molding tube.

The analysis apparatus 100 of this embodiment may be adapted to analyze as a first analysis step free carbon in a specimen with the internal temperature of the first heating furnace 1a in the second temperature range, and then, after the first analysis is complete, analyze as a second analysis step carbon other than free carbon in the specimen in the second heating furnace 1b in the first temperature range. Total carbon in the specimen may be analyzed by using the analyzing results in the first heating furnace 1a and in the second heating furnace 1b.

In such a configuration, since the furnace body 12a is a silica tube, adsorption of free carbon to the furnace body 12a can be efficiently prevented, and precision of analysis for free carbon can be improved.

Further, since the furnace bodies 12a and 12b are used in a proper temperature range for their material, cracking of the furnace bodies 12a and 12b can be prevented.

When analyzing free carbon with the internal temperature of a heating furnace in the second temperature range, the time required to combust a specimen and to retain analysis target gas in a gas lead-out flow path 31 is made longer than the time required to retain analysis target gas from a normal specimen. In doing so, the amount of analysis target gas led out to the analyzer 2 per unit time can be increased to improve an S/N ratio. As a result, free carbon can be accurately analyzed.

When measuring free carbon in such a configuration, in terms of analysis efficiency, it is preferable to switch on-off valves from the closed state to the opened state within 10 minutes after starting to combust a specimen.

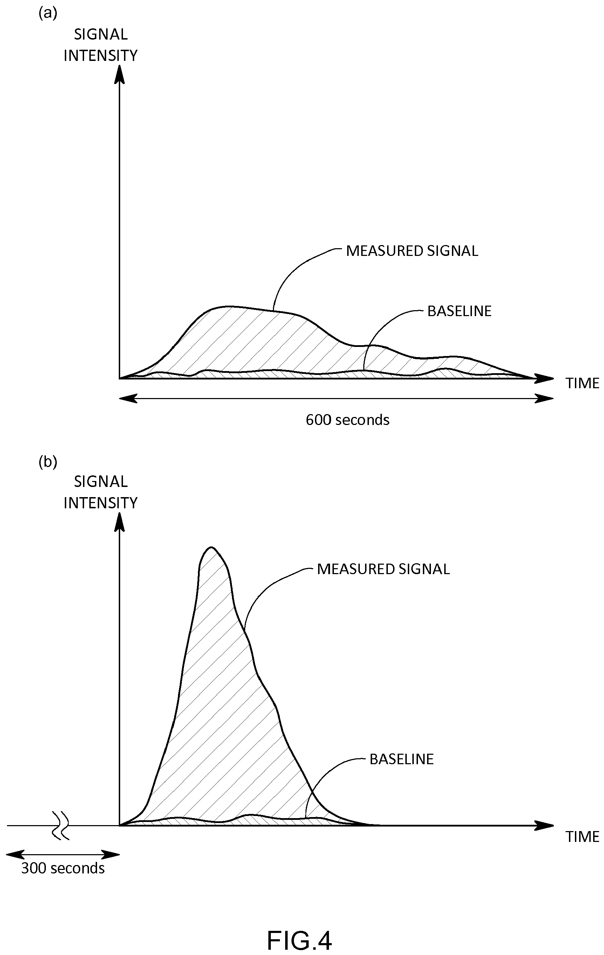

FIG. 4 illustrates examples of a variation in signal intensity with respect to time obtained when a specimen was combusted with the internal temperature of a heating furnace in the second temperature range and analysis target gas derived from free carbon was analyzed.

FIG. 4(a) illustrates an output result obtained by leading out produced analysis target gas to the analyzer 2 without retaining upstream of an on-off valve 32. On the other hand, FIG. 4(b) illustrates an output result obtained by, without promptly leading out the produced analysis target gas to the analyzer 2, retaining the analysis target gas upstream of the on-off valve 32, and after approximately 300 seconds since the start of combustion, bringing the on-off valve 32 into the opened state to lead out the analysis target gas to the analyzer 2. When comparing FIGS. 4(a) and 4(b), it turns out that by retaining the produced analysis target gas without promptly leading out it to the analyzer 2, the resulting signal intensity is increased, and the S/N ratio is improved to improve the accuracy of measuring free carbon.

In the above-described embodiment, each of the dust removing mechanisms is configured such that the first horizontal pipe part and the second horizontal pipe part are coaxial, and the inside diameter of the second horizontal pipe part is larger than the inside diameter of the first horizontal pipe part, but is not limited to this. As long as the concave part can be formed below each of the filters 62, the second horizontal pipe part may be of another shape. The second horizontal pipe part may be, for example, formed with a concave part 19 only on the lower side of the filter 62, and connected to and flush with the first horizontal pipe part in the other part.

The analysis apparatus 100 of the above-described embodiment is one including the two heating furnaces, i.e., the first heating furnace 1a and the second heating furnace 1b, but may be one including more furnaces. Even in such a case, the above-described effects can be obtained by including a selecting mechanism 3 adapted to selectively communicatively connect each of the heating furnaces to the analyzer 2.

Also, an analysis apparatus 100 of another embodiment may be one having only one heating furnace. In this case, the analysis apparatus 100 may be configured to provide one flow path with the functions as the combustion control mechanism 7 and the combustion supporting gas heating mechanism 8.

Further, an analysis apparatus 100 of another embodiment may be configured not to include a combustion supporting gas heating mechanism 8 but to include a gas purifier and remove hydrocarbons in combustion supporting gas through the gas purifier. In this case, the analysis apparatus 100 may be configured not to provide combustion control mechanisms 7a and 7b with heating means 73a and 73b, respectively.

The analysis apparatus 100 of the above-described embodiment is one adapted to supply the combustion supporting gas such as oxygen into the heating furnaces, but not limited to this.

The analysis apparatus 100 of the above-described embodiment is one such that the first heating furnace 1a and the second heating furnace 1b respectively include: the furnace bodies for containing the vessels; and the electric resistors for generating heat by current application to heat the furnace bodies, but not limited to this. In addition to the configuration adapted to provide the dust removing mechanisms respectively just after the gas lead-out ports of the heating furnaces, furnaces of a type called high frequency heating furnaces may be used as the heating furnaces.

In the above-described embodiment, when a specimen is deflagrative, after a predetermined time has passed since the specimen started to combust, the flow rate of the combustion supporting gas to be supplied to the heating furnace 1a (or 1b) may be increased by operating the flow rate regulation valve 72a (or 72b) in a closing direction to decrease the flow rate of the combustion supporting gas flowing through the bypass flow path 71a (or 71b). In doing so, the combustion of the specimen is facilitated, and consequently the time required for analysis can be shortened.

In the above-described embodiment, the heating means 82 is one adapted to heat the combustion supporting gas using heat from the heater and the like attached on the outer circumference of the branch flow path 81, but not limited to this. In another embodiment, heating means 82 may be one adapted to heat combustion supporting gas flowing through a branch flow path 81 using heat generated by an electric resistor 14a (or 14b) of a heating furnace 1a (or 1b). Such a configuration can be achieved by, for example, passing a part of the branch flow path 81 through the inside of a heat insulating material that is near the electric resistor 14a (or 14b) and covers the electric resistor 14a (or 14b). This configuration makes it possible to more reduce cost and size because it is not necessary to separately provide a heater for heating the branch flow path 81.

The heating means 73a and 73b may also be configured to heat the combustion supporting gas flowing through the bypass flow paths 71a and 71b using heat generated by the electric resistors 14a and 14b of the heating furnaces 1a and 1b, respectively.

The heating furnace selecting mechanism 3 in the above-described embodiment is one including the on-off valves 32a and 32b, which are two way valves for opening/closing the flow paths, in the first gas lead-out flow path 31a and in the second gas lead-out flow path 31b, but not limited to this. Another embodiment may be configured to include three-way valves respectively for opening/closing a first gas lead-out flow path 31a and a second gas lead-out flow path 31b.

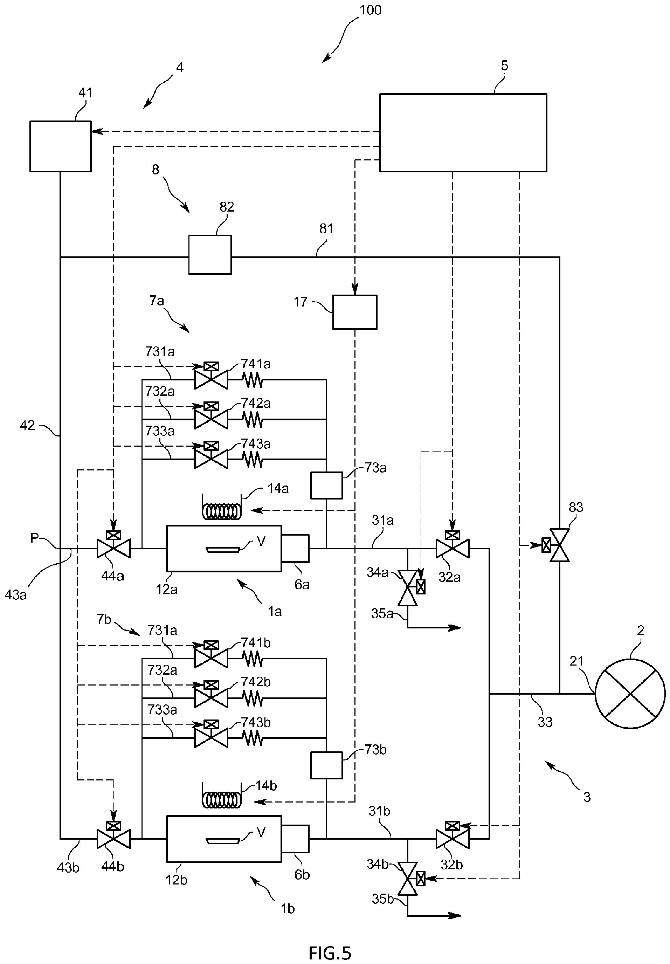

Further, as illustrated in FIG. 5, in an analysis apparatus 100 of another embodiment, a bypass flow path of a combustion control mechanism 7a may be one branching into multiple branch bypass flow paths 731a to 733a that are provided in parallel and respectively have predetermined resistances. The branch bypass flow paths 731a to 733a may be formed of resistors having mutually different resistances, such as capillary tubes whose diameters and lengths are different.

In this embodiment, opening level regulating means is one including on-off valves 741a to 743a respectively provided in the branch bypass flow paths 731a to 733a. These valves can be opened/closed by signals from a control part 5. By regulating an opened/closed state of each of the on-off valves 741a to 743a, the flow rate of combustion supporting gas to be supplied to a first heating furnace 1a can be more finely regulated in multiple steps.

A combustion control mechanism 7b may be configured in the same manner.

In addition, in the above-described embodiment, the end points of the bypass flow paths 71a and 71b are connected to the gas lead-out flow paths 31a and 31b, respectively, but not limited to this. In another embodiment, without being connected to gas lead-out flow paths 31a and 31b, the end point of bypass flow paths 71a and 71b may be opened to the outside, so that combustion supporting gas flowing through the bypass flow paths 71a and 71b is discharged to the outside. Note that in this case, the flow rate of the gas flowing through an analyzer 2 is not substantially constant.

Besides, it should be appreciated that the present invention is not limited to any of the above-described embodiments and various modifications including appropriate combinations of parts of the configurations of the respective illustrated embodiments can be made without departing from the scope thereof.

### Reference Signs List

100: Analysis apparatus
1: Heating furnace
1a: First heating furnace
1b: Second heating furnace
2: Analyzer
3: Heating furnace selecting mechanism
7: Combustion control mechanism
8: Combustion supporting gas heating mechanism
V: Vessel

## Claims

1. An analysis apparatus comprising: a vessel for containing a specimen; a heating furnace to be loaded with the vessel; and an analyzer that analyzes analysis target gas produced from the specimen heated and combusted in the heating furnace, the analysis apparatus comprising:
a combustion supporting gas supply flow path for supplying combustion supporting gas used for the combustion of the specimen to the heating furnace; and
a combustion control mechanism adapted to regulate a flow rate of the combustion supporting gas to be supplied to the heating furnace from the combustion supporting gas supply flow path and control the combustion of the specimen.

2. The analysis apparatus according to claim 1, wherein
the combustion control mechanism comprises:
a bypass flow path branching from the combustion supporting gas supply flow path; and
opening level regulating means adapted to regulate an opening level of the bypass flow path, and
is one adapted to regulate the flow rate of the combustion supporting gas to be supplied to the heating furnace by flowing part of the combustion supporting gas flowing through the combustion supporting gas supply flow path to the bypass flow path.

3. The analysis apparatus according to claim 2, wherein
the bypass flow path is one including multiple branch bypass flow paths that are provided in parallel and respectively have predetermined resistances, and
the opening level regulating means is one including on-off valves respectively provided in the branch bypass flow paths.

4. The analysis apparatus according to claim 2 or 3, wherein
the bypass flow path is provided in parallel with the heating furnace and communicatively connects to the analyzer.

5. The analysis apparatus according to any of claims 2 to 4, further comprising:
a branch flow path branching from the combustion supporting gas supply flow path:
a shut-off valve that shuts off the combustion supporting gas introduced to the heating furnace to wholly introduce the combustion supporting gas flowing through the combustion supporting gas supply flow path to the branch flow path; and
heating means adapted to heat the combustion supporting gas flowing through the branch flow path, wherein
an offset correction value for the analyzer is determined with use of an output result of the analyzer in a state where the shut-off valve operates and the combustion supporting gas is wholly introduced into the analyzer through the branch flow path.

6. The analysis apparatus according to any of claims 1 to 5, wherein the heating furnace is one including: a furnace body for containing the vessel; and an electric resistor for generating heat by current application to heat the furnace body.

7. The analysis apparatus according to any of claims 1 to 6, wherein
the heating furnace is one having a gas lead-out port for leading out the analysis target gas to the analyzer,
the analysis apparatus further comprising a dust removing mechanism attached continuous with the gas lead-out port, wherein
the dust removing mechanism is one including a filter for collecting dust, and a casing for holding the filter inside, and
the casing has a horizontal pipe part adapted to circulate the analysis target gas in a horizontal direction and hold the filter, and includes a concave part below the filter in the horizontal pipe part.

8. The analysis apparatus according to any of claims 1 to 7, comprising:
multiple heating furnaces; and
a heating furnace selecting mechanism adapted to selectively communicatively connect any of the heating furnaces to the analyzer.

9. The analysis apparatus according to claim 8, wherein
the heating furnace selecting mechanism is one comprising:
multiple gas lead-out flow paths whose start points communicatively connect to the respective heating furnaces and whose end points communicatively connect to the analyzer; and
on-off valves for opening/closing the respective gas lead-out flow paths.

10. The analysis apparatus according to claim 8 or 9,
making the multiple heating furnaces loaded with vessels containing specimens simultaneously perform heating operation, and making the heating furnace selecting mechanism communicatively connect only one of the furnaces to the analyzer.

11. The analysis apparatus according to claim 9, wherein
on upper stream sides than the on-off valves in the gas lead-out flow paths, openable/closable gas discharge flow paths for discharging gas flowing through the gas lead-out flow paths are connected.

12. The analysis apparatus according to any of claims 1 to 11, wherein
internal temperature of the heating furnace is changeable between a predetermined first temperature range and a second temperature range lower than the first temperature range, and
in a state where the internal temperature is in the second temperature range, an on-off valve in a gas lead-out flow path of the heating furnace is switched from a closed state to an opened state after a predetermined time has passed since the specimen was loaded into the heating furnace.

13. An analysis method that in a heating furnace, combusts a specimen contained in a vessel, and with use of an analyzer, analyzes analysis target gas resulting from the combustion, the analysis method comprising:
heating combustion supporting gas used to combust the specimen;
introducing the heated combustion supporting gas into the analyzer; and
determining an offset correction value for the analyzer with use of an output result of the analyzer in a state where the heated combustion supporting gas is introduced.
